# EUROPEAN PATENT SPECIFICATION

(11) **EP 3 547 989 B1**
(45) Date of publication and mention of the grant of the patent: **05.10.2022**
(21) Application number: 17794299.2
(22) Date of filing: 30.10.2017
(51) Int. Cl.: A61K 8/24, A61Q 11/00, A61K 8/19

(54) **ORAL CARE COMPOSITION**
MUNDPFLEGEZUSAMMENSETZUNG
COMPOSITION DE PROTECTION ORALE

(30) Priority: 30.11.2016 WO PCT/CN2016/107981; 03.01.2017 EP 17150152
(43) Date of publication of application: 09.10.2019
(73) Proprietor: Unilever Global IP Limited, Wirral, Merseyside CH62 4ZD (GB); Unilever IP Holdings B.V., 3013 AL Rotterdam (NL)
(72) Inventor: LI, Xiaoke, Shanghai 200335 (CN); LIU, Weining, Shanghai 200335 (CN); WANG, Jinfang, Shanghai 200335 (CN)
(74) Representative: Tansley, Sally Elizabeth
(86) International application number: PCT/EP2017/077778
(87) International publication number: WO 2018/099669

(56) References cited:
- WO-A1-2017/005431
- US-A1- 2016 193 118
- DATABASE GNPD [Online] MINTEL; 1 December 2011 (2011-12-01), ANONYMOUS: "Anti-Sensitivity and Gum Protecting Toothpaste", XP002768336, Database accession no. 1661761
- ANONYMOUS: "Anti-Sensitivity and Gum Protecting Toothpaste", GNPD, MINTEL, 1 December 2011 (2011-12-01), XP002768336,

## Description

### Technical Field of the Invention

The present invention relates to oral care compositions such as tooth pastes, powders, gums, mouthwashes and the like. In particular the present invention relates to an oral care composition comprising a water insoluble and/or slightly soluble calcium source, a tubule blockage enhancer and a phosphate source that reduces tooth sensitivity. This invention also relates to such compositions for use in treating tooth hypersensitivity and/or remineralizing teeth of an individual.

### Background of the Invention

Tooth hypersensitivity is a temporary induced pain sensation that affects up to 20% of the adult population. Hypersensitive teeth may be sensitive to temperature, pressure or chemical action.

The dentin of the tooth generally contains channels, called tubules, which provide for an osmotic flow between the inner pulp region of the tooth and the outer root surfaces. Tooth hypersensitivity may be related to the general increase in exposed root surfaces of teeth as a result of periodontal disease, toothbrush abrasion, or cyclic loading fatigue of the thin enamel near the dentin-enamel junction. When root surfaces are exposed, dentinal tubules are also exposed.

The currently accepted theory for tooth hypersensitivity is the hydrodynamic theory, based on the belief that open exposed dentinal tubules allow fluid flow through the tubules. This flow excites the nerve endings in the dental pulp. Clinical replica of sensitive teeth viewed in a SEM (scanning electron microscopy) reveal varying numbers of open or partially occluded dentinal tubules.

There are different approaches to treat tooth hypersensitivity. One approach is to reduce the excitability of the nerve in a sensitive tooth by using "nerve-depolarising agents" comprising strontium ions, potassium salts such as potassium nitrate, potassium bicarbonate, potassium chloride and the like. These nerve-depolarising agents function by interfering with neural transduction of the pain stimulus to make the nerve less sensitive.

Another approach is to use "tubule blocking agents" that fully or partially occlude tubules such as polystyrene beads, apatite, polyacrylic acid, mineral hectorite clay and the like. These tubule blocking agents function by physically blocking the exposed ends of the dentinal tubules, thereby reducing dentinal fluid movement and reducing the irritation associated with the shear stress described by the hydrodynamic theory.

It has always been of great interest to treat tooth hypersensitivity with a more efficient approach. The present inventor have now found unexpectedly that an oral care composition comprising a water insoluble and/or slightly soluble calcium source, a tubule blockage enhancer and a phosphate source provides excellent tubule blockage efficacy to reduce tooth sensitivity. Additionally, such a composition can enhance the tooth remineralization efficacy and/or the deposition of benefit agents on tooth surfaces to further benefit teeth of an individual.

### Additional Information

WO 2008/068149 A (Unilever) discloses an oral care product comprising a first composition comprising an insoluble calcium salt that is not a calcium phosphate salt, a second independent composition comprising a source of phosphate ions, and a means for delivering each of the compositions to the surface of the teeth. The preferred insoluble calcium salt is calcium silicate.

US 2016/0193118 A1 (Kuraray Noritake Dental INC.) discloses a dentinal tubule sealing material excellent in durability of dentinal tubule sealing performance (acid resistance) and also in storage stability.

Database GNPD Mintel, "Anti-sensitivity and gum protecting toothpaste", XP002768336, database accession no. 1661761 discloses an anti-sensitivity and gum protecting toothpaste containing active ingredients extracted from expensive panax pseudoginseng.

The publication above does not describe an oral care composition comprising a water insoluble and/or slightly soluble calcium source, a tubule blockage enhancer selected from calcium dihydrogen phosphate, calcium sulfate hemihydrate or mixtures thereof, a phosphate source and a physiologically acceptable carrier; wherein the calcium source is selected from calcium carbonate, calcium aluminate, calcium oxalate, aluminium calcium silicate, calcium hydroxide, calcium glycerophosphate, calcium oxide, calcium sulfate, calcium carboxymethyl cellulose, calcium alginate, calcium salts of citric acid or mixtures thereof, wherein the phosphate source is trisodium phosphate, monosodium dihydrogen phosphate, disodium hydrogen phosphate, ammonium phosphate, diammonium hydrogen phosphate, ammonium dihydrogen phosphate, tripotassium phosphate, monopotassium dihydrogen phosphate, dipotassium hydrogen phosphate or a mixture thereof; and wherein the calcium source and the tubule blockage enhancer are present in a weight ratio from 20:1 to 1:5, and especially such an oral care composition can treat tooth hypersensitivity and/or enhance tooth remineralization efficacy.

### Tests and Definitions

### Dentifrice

"Dentifrice" for the purposes of the present invention means a paste, powder, liquid, gum or other preparation for cleaning the teeth or other surfaces in the oral cavity.

### Tooth Paste

"Tooth paste" for the purpose of the present invention means a paste or gel dentifrice for use with a toothbrush. Especially preferred are tooth pastes suitable for cleaning teeth by brushing for about two minutes.

### Particle Size

"Particle size" for the purpose of the present invention means D50 particle size. The D50 particle size of a particulate material is the particle size diameter at which 50 wt% of the particles are larger in diameter and 50 wt% are smaller in diameter. For the purpose of the present invention, particle sizes and distribution of the water insoluble and/or slightly soluble calcium source are using Sedigraph Series 51 through sedimentation analysis. The sample to be analysed is introduced as a suspension into the sedimentation cell through a peristaltic pump. A finely collimated beam of X-ray passes through the suspended medium. The concentration of sediment in the beam is proportional to the X-ray intensity. The software of the instrument solves stokes' Law and presents the final result linearly as a cumulative mass percent distribution.

### Composite Particle

"Composite particle" for the purpose of the present invention means a particle comprising a first component core and a second component coating wherein the core and coating are composed of different materials.

### Refractive Index

Refractive index is quoted at a temperature of 25°C and a wavelength of 589 nm.

### Tubule Blockage Enhancer

"Tubule blockage enhancer" for the purpose of the present invention means a material which aids the deposition of actives such as water insoluble and/or slightly soluble calcium source from the continuous phase of the composition onto dentine and/or into dentinal tubules to induce *in situ* generation of calcium phosphate that occludes the dentinal tubules and/or their exposed open ends. When benefit agents are present in the oral care composition, the remineralization of the calcium source around the benefit agents further helps the retention of those benefit agents on tooth surfaces by enhancing their resistance to shear force. Benefit agents as used herein mean an active typically delivered to human teeth and/or the oral cavity including the gums to enhance or improve a characteristic of those dental tissues.

### pH

pH is quoted at atmospheric pressure and a temperature of 25°C. When referring to the pH of an oral care composition, this means the pH measured when 5 parts by weight of the composition is uniformly dispersed and/or dissolved in 20 parts by weight pure water at 25°C. In particular the pH may be measured by manually mixing 5 g oral care composition with 20 mL water for 30 s, then immediately testing the pH with indicator or a pH meter.

### Solubility

"Soluble" and "insoluble" for the purpose of the present invention means the solubility of a source (e.g., like calcium salts) in water at 25°C and atmospheric pressure. "Soluble" means a source that dissolves in water to give a solution with a concentration of at least 0.1 moles per litre. "Insoluble" means a source that dissolves in water to give a solution with a concentration of less than 0.001 moles per litre. "Slightly soluble", therefore, is defined to mean a source that dissolves in water to give a solution with a concentration of greater than 0.001 moles per litre and less than 0.1 moles per litre.

### Substantially Free

"Substantially free of' for the purpose of the present invention means less than 3.0%, and preferably less than 2.0%, and more preferably less than 1.0% and most preferably less than 0.5% by weight, based on total weight of the oral care composition, including all ranges subsumed therein.

### Dual-Phase

"Dual-Phase" for the purpose of the present invention means a composition having two independent phases which are physically separate.

### Anhydrous Composition

"Anhydrous composition" for the purpose of the present invention means the water content of the composition is less than 3.0%, and preferably less than 2.0%, and more preferably less than 1.0% and most preferably less than 0.5% by total weight of the oral care composition.

### Viscosity

Viscosity of a tooth paste is the value taken at room temperature (25 °C) with a Brookfield Viscometer, Spindle No.4 and at a speed of 5 rpm. Values are quoted in centipoises (cP=mPa.s) unless otherwise specified.

### Remineralization

"Remineralization" for the purpose of the present invention means *in situ* (i.e. in the oral cavity) generation of calcium phosphate on teeth (including layers on teeth from 10 nm to 20 microns, and preferably from 75 nm to 10 microns, and most preferably, from 150 nm to 5 microns thick including all ranges subsumed therein) to reduce the likelihood of tooth sensitivity, tooth decay, regenerate enamel and/or improve the appearance of teeth by whitening through the generation of such new calcium phosphate.

### Miscellaneous

Except in the examples, or where otherwise explicitly indicated, all numbers in this description indicating amounts of material or conditions of reaction, physical properties of materials and/or use may optionally be understood as modified by the word "about".

All amounts are by weight of the final oral care composition, unless otherwise specified.

It should be noted that in specifying any ranges of values, any particular upper value can be associated with any particular lower value.

For the avoidance of doubt, the word "comprising" is intended to mean "including" but not necessarily "consisting of" or "composed of". In other words, the listed steps or options need not be exhaustive.

The disclosure of the invention as found herein is to be considered to cover all embodiments as found in the claims as being multiply dependent upon each other irrespective of the fact that claims may be found without multiple dependency or redundancy.

Where a feature is disclosed with respect to a particular aspect of the invention (for example a composition of the invention), such disclosure is also to be considered to apply to any other aspect of the invention (for example a method of the invention) *mutatis mutandis.*

### Summary of the Invention

In a first aspect, the present invention is directed to an oral care composition comprising:
a) a water insoluble and/or slightly soluble calcium source;
b) a tubule blockage enhancer selected from calcium dihydrogen phosphate, calcium sulfate hemihydrates or mixtures thereof;
c) a phosphate source; and
d) a physiologically acceptable carrier;
wherein the calcium source is selected from calcium carbonate, calcium aluminate, calcium oxalate, aluminium calcium silicate, calcium hydroxide, calcium glycerophosphate, calcium oxide, calcium sulfate, calcium carboxymethyl cellulose, calcium alginate, calcium salts of citric acid or mixtures thereof or mixtures thereof; wherein the phosphate source is trisodium phosphate, monosodium dihydrogen phosphate, disodium hydrogen phosphate, ammonium phosphate, diammonium hydrogen phosphate, ammonium dihydrogen phosphate, tripotassium phosphate, monopotassium dihydrogen phosphate, dipotassium hydrogen phosphate or a mixture thereof; and wherein the calcium source and the tubule blockage enhancer are present in a weight ratio from 20:1 to 1:5.

In a second aspect, the present invention is directed to a packaged oral care product comprising the oral care composition of the first aspect of this invention.

In a third aspect, the present invention is directed to an oral care composition for use in reducing sensitivity and/or remineralization of teeth of an individual comprising the step of applying the oral care composition of any embodiment of the first aspect to at least one surface of the teeth of the individual.

All other aspects of the present invention will more readily become apparent upon considering the detailed description and examples which follow.

### Detailed Description

Now it has been found that an oral care composition comprising a water insoluble and/or slightly soluble calcium source, a tubule blockage enhancer and a phosphate source provides excellent tubule blockage efficacy to reduce tooth sensitivity. Additionally, such a composition can also enhance the tooth remineralization efficacy and/or the deposition of benefit agents on tooth surfaces to further benefit teeth of an individual.

The water insoluble and slightly soluble calcium source suitable for use in this invention is limited only to the extent that the same may be used in the mouth. Typically, the calcium source has a D50 particle size of 5 micron or less, more preferably from 0.01 to 3 microns, and most preferably from 0.01 to 1.5 microns. In an especially preferred embodiment, from 1 to 100% by weight of the calcium source used in this invention has a D50 particle size from 0.01 to 1.5 microns.

Illustrative yet non-limiting examples of the types of calcium source that may be used in the invention include, for example, calcium carbonate, calcium aluminate, calcium oxalate, aluminium calcium silicate, calcium hydroxide, calcium glycerophosphate, calcium oxide, calcium sulfate, calcium carboxymethyl cellulose, calcium alginate, calcium salts of citric acid or mixtures thereof or mixtures thereof. In a preferred embodiment, the calcium source comprises at least 80% by weight of calcium carbonate based on total weight of the calcium source. Most preferably, the calcium source comprises from 90 to 100% by weight of calcium carbonate or is calcium carbonate. Such calcium carbonate suitable for use in this invention are commercially available, for example, from Omya under the trade name OMYACARE HP 900 OG.

Typically, the oral care composition of the present invention comprises from 0.1 to 80% by weight of the water insoluble and/or slight soluble calcium source, more preferably from 0.2 to 50%, most preferably from 1 to 30% based on the total weight of the oral care composition and including all ranges subsumed therein. In a preferred embodiment, the oral care composition of the present invention comprises from 0.1 to 80% by weight of the water insoluble and/or slightly soluble calcium source having a D50 particle size of 5 microns or less, more preferably from 0.2 to 50%, and most preferably from 1 to 30% based on the total weight of the oral care composition and including all ranges subsumed therein.

The water insoluble and/or slightly soluble calcium source having a D50 particle size of greater than 5 microns may be employed in this invention along with the calcium source described herein. In an optional embodiment, the oral care composition of the present invention can comprise from 0.01 to 45%, and preferably from 0.1 to 40%, and most preferably from 5 to 35% by weight of the water insoluble and/or slightly soluble calcium source having a particle size greater than 5 microns and including all ranges subsumed therein. It is within the scope of the present invention for the oral care composition to comprise from 1 to 10% by weight of the water insoluble and/or slightly soluble calcium source like calcium carbonate having a particle size greater than 5 microns and it is further within the scope of the present invention for the oral care composition to be free of the water insoluble and/or slightly soluble calcium source having a particle size of greater than 5 microns.

The tubule blockage enhancer suitable for use in this invention is limited only to the extent that the same may be used in the mouth. In a preferred embodiment, the tubule blockage enhancer provides plenty of calcium ions in the mouth and is inexpensive and abundant. In an especially preferred embodiment, the tubule blockage enhancer is calcium salts in addition to the water insoluble and/or slightly soluble calcium source which is included in the composition.

It has been discovered that the materials used as tubule blockage enhancer in this invention is biocompatible, and undergoes rapid reaction in water and complete resorption onto the dentine and/or enamel of teeth, therefore it can be used to aid the deposition of oral care actives such as water insoluble and/or slightly soluble calcium source onto dentine and/or into dentinal tubules to induce *in situ* generation of calcium phosphate that occludes the dentinal tubules and/or their exposed open ends.

Illustrative yet non-limiting examples of the types of tubule blockage enhancer that may be used in this invention include, for example, calcium sulfate hemihydrates, calcium dihydrogen phosphate, calcium monohydrogen phosphate, mixtures thereof or the like. In an especially preferred embodiment, the tubule blockage enhancer is calcium sulfate hemihydrates, calcium dihydrogen phosphate or mixtures thereof. Most preferably, the tubule blockage enhancer is calcium dihydrogen phosphate.

Typically, the oral care composition of the present invention comprises from 0.01 to 20% by weight of the tubule blockage enhancer, more preferably from 0.1 to 15%, more preferably still from 0.1 to 10%, most preferably from 0.2 to 5%, based on the total weight of the oral care composition and including all ranges subsumed therein.

The oral care composition preferably comprises the water insoluble and/or slightly soluble calcium source and the tubule blockage enhancer in a weight ratio from 20:1 to 1:5, more preferably from 15:1 to 1:3, most preferably from 10:1 to 1:1.

The phosphate source that may be used in this invention is limited only to the extent that the same may be used in a composition suitable for use in the mouth. The phosphate source is able to provide phosphate ions to react with the water insoluble and/or slightly soluble calcium source to produce a calcium phosphate *in situ* reaction product that is a precursor for hydroxyapatite formation.

Illustrative examples of the types of phosphate source suitable for use in this invention include trisodium phosphate, monosodium dihydrogen phosphate, disodium hydrogen phosphate, ammonium phosphate, diammonium hydrogen phosphate, ammonium dihydrogen phosphate, tripotassium phosphate, monopotassium dihydrogen phosphate, dipotassium hydrogen phosphate, mixtures thereof or the like. The phosphate source is preferably one which is water soluble.

When used, the phosphate source typically makes up from 0.1 to 40%, and more preferably, from 0.5 to 30%, and most preferably, from 1 to 20% by weight of the oral care composition, based on total weight of the oral care composition and including all ranges subsumed therein. In a preferred embodiment, the phosphate source used is trisodium phosphate and monosodium dihydrogen phosphate at a trisodium phosphate to monosodium dihydrogen phosphate weight ratio of 1:4 to 4:1, preferably 1:3 to 3:1, and most preferably, from 1:2 to 2:1, including all ratios subsumed therein. In another preferred embodiment, the phosphate source used is or at least comprises monosodium dihydrogen phosphate. In another especially preferred embodiment, the phosphate source is monopotassium dihydrogen phosphate, dipotassium hydrogen phosphate or a mixture thereof.

The oral care composition preferably comprises the water insoluble and/or slightly soluble calcium source and the phosphate source in a weight ratio from 10:1 to 1:5, more preferably from 5:1 to 1:3, most preferably from 3:1 to 1:1.

The composition of the present invention is an oral care composition and typically comprises a physiologically acceptable carrier. The carrier preferably comprises at least surfactant, thickener, humectant or a combination thereof.

Preferably the oral care composition comprises a surfactant. Preferably the composition comprises at least 0.01% surfactant by weight of the composition, more preferably at least 0.1% and most preferably from 0.5 to 7%. Suitable surfactants include anionic surfactants, such as the sodium, magnesium, ammonium or ethanolamine salts of Cs to C₁₈ alkyl sulphates (for example sodium lauryl sulphate), C₈ to C₁₈ alkyl sulphosuccinates (for example dioctyl sodium sulphosuccinate), C₈ to C₁₈ alkyl sulphoacetates (such as sodium lauryl sulphoacetate), C₈ to C₁₈ alkyl sarcosinates (such as sodium lauryl sarcosinate), C₈ to C₁₈ alkyl phosphates (which can optionally comprise up to 10 ethylene oxide and/or propylene oxide units) and sulphated monoglycerides. Other suitable surfactants include nonionic surfactants, such as optionally polyethoxylated fatty acid sorbitan esters, ethoxylated fatty acids, esters of polyethylene glycol, ethoxylates of fatty acid monoglycerides and diglycerides, and ethylene oxide/propylene oxide block polymers. Other suitable surfactants include amphoteric surfactants, such as betaines or sulphobetaines. Mixtures of any of the above described materials may also be used. More preferably the surfactant comprises or is anionic surfactant. The preferred anionic surfactants are sodium lauryl sulphate and/or sodium dodecylbenzene sulfonate. Most preferably the surfactant is sodium lauryl sulphate, sodium coco sulfate, cocamidopropyl betaine, sodium methyl cocoyl taurate or mixtures thereof.

Thickener may also be used in this invention and is limited only to the extent that the same may be added to a composition suitable for use in the mouth. Illustrative examples of the types of thickeners that may be used in this invention include, sodium carboxymethyl cellulose (SCMC), hydroxyl ethyl cellulose, methyl cellulose, ethyl cellulose, gum tragacanth, gum arabic, gum karaya, sodium alginate, carrageenan, guar, xanthan gum, Irish moss, starch, modified starch, silica based thickeners including silica aerogels, magnesium aluminum silicate (e.g., Veegum), Carbomers (cross-linked acrylates) and mixtures thereof.

Typically, xanthan gum and/or sodium carboxymethyl cellulose and/or a Carbomer is/are preferred. When a Carbomer is employed, those having a weight-average molecular weight of at least 700,000 are desired, and preferably, those having a molecular weight of at least 1,200,000, and most preferably, those having a molecular weight of at least about 2,500,000 are desired. Mixtures of Carbomers may also be used herein.

In an especially preferred embodiment, the Carbomer is Synthalen PNC, Synthalen KP or a mixture thereof. It has been described as a high molecular weight and cross-linked polyacrylic acid and identified via CAS number 9063-87-0. These types of materials are available commercially from suppliers like Sigma.

In another especially preferred embodiment, the sodium carboxymethyl cellulose (SCMC) used is SCMC 9H. It has been described as a sodium salt of a cellulose derivative with carboxymethyl groups bound to hydroxy groups of glucopyranose backbone monomers and identified via CAS number 9004-32-4. The same is available from suppliers like Alfa Chem.

In another especially preferred embodiment, the thickener is xanthan gum.

Thickener typically makes up from 0.01 to about 10%, more preferably from 0.1 to 9%, and most preferably, from 0.1 to 5% by weight of the oral care composition, based on total weight of the composition and including all ranges subsumed therein.

When the oral care composition of this invention is a toothpaste or gel, the same typically has a viscosity from about 30,000 to 180,000 centipoise, and preferably, from 60,000 to 170,000 centipoise, and most preferably, from 65,000 to 165,000 centipoise.

Suitable humectants are preferably used in the oral care composition of the present invention and they include, for example, glycerin, sorbitol, propylene glycol, dipropylene glycol, diglycerol, triacetin, mineral oil, polyethylene glycol (preferably, PEG-400), alkane diols like butane diol and hexanediol, ethanol, pentylene glycol, or a mixture thereof. Glycerin, polyethylene glycol, sorbitol or mixtures thereof are the preferred humectants.

The humectant may be present in the range of from 10 to 90% by weight of the oral care composition. More preferably, the carrier humectant makes up from 25 to 80%, and most preferably, from 30 to 60% by weight of the composition, based on total weight of the composition and including all ranges subsumed therein.

The oral care composition may further comprise benefit agents that are typically delivered to human teeth and/or the oral cavity including the gums to enhance or improve a characteristic of those dental tissues. The only limitation with respect to the benefit agents that may be used in this invention is that the same is suitable for use in the mouth.

Typically the benefit agent is selected from optical agents, biomineralization agents, antibacterial agents, gum health agents, desensitizing agents, anti-calculus agents, freshness agents or mixtures thereof. Preferably, the benefit agent is selected from optical agents, biomineralization agents, antibacterial agents, gum health agents, freshness agents or mixtures thereof.

For example, optical agents such as coloring agents like whitening agents and pigments. Preferably, the pigment, when used, is violet or blue having a hue angle, h, in the CIELAB system of from 220 to 320 degrees. These pigments may be selected from one or more of those listed in the Colour Index International, listed as pigment blue 1 through to pigment blue 83, and pigment violet 1 through to pigment violet 56. In another preferred embodiment, the optical agents may be selected from one or more of mica, interference mica, boron nitride, poly(methyl methacrylate) flake, composite microspheres, titanium dioxide coated glass flake, inverse opal, cholesteric liquid crystal, photonic sphere, hollow sphere and zinc oxide. Biomineralization agents for tooth enamel remineralization may be selected from one or more of fluoride sources, biomolecules, proteinaceous materials, amorphous calcium phosphate, α-tricalcium phosphate, β-tricalcium phosphate, calcium deficient hydroxyapatite Ca_{10-X}(HPO₄)_{X}(PO₄)_{6-X}(OH)_{2-X}, 0 ≤ x < 1), dicalcium phosphate (CaHPO₄), dicalcium phosphate dihydrate (CaHPO₄·2H₂O), hydroxyapatite (Ca₁₀(PO₄)₆(OH)₂), monocalcium phosphate monohydrate (Ca(H₂PO₄)₂·H₂O), octacalcium phosphate (Ca₈H₂(PO₄)₆·5H₂O) and tetracalcium phosphate (Ca₄(PO₄)₂O). Antibacterial agents may be selected from one or more of metal salts where the metal is selected from zinc, copper, silver or a mixture thereof, triclosan, triclosan monophosphate, triclocarban, curcumin, quaternary ammonium compounds, bisbiguanides and long chain tertiary amines, preferably zinc salts including zinc oxide, zinc chloride, zinc acetate, zinc ascorbate, zinc sulphate, zinc nitrate, zinc citrate, zinc lactate, zinc peroxide, zinc fluoride, zinc ammonium sulfate, zinc bromide, zinc iodide, zinc gluconate, zinc tartarate, zinc succinate, zinc formate, zinc phenol sulfonate, zinc salicylate, zinc glycerophosphate or a mixture thereof. Gum health agents may be selected from one or more of anti-inflammatory agents, plaque buffers, biomolecules, proteinaceous materials, vitamin, plant extracts and curcumin. Freshness agents may be flavors selected from one or more of peppermint, spearmint, menthol, flora oil, clove oil and citrus oil.

The benefit agent is preferably particulate as this allows for maximum surface area for contact with dental tissue.

In a preferred embodiment, the benefit agent is a particulate whitening agent for tooth whitening.

Typically, the particulate whitening agent comprises a material suitable to physically and immediately improve characteristics of teeth and especially whiten teeth. In order to provide excellent whitening effect, the material is preferred to have a high refractive index of at least 1.9, more preferably at least 2.0, even more preferably at least 2.2, even more preferably still at least 2.4 and most preferably at least 2.5. The maximum refractive index of the material is not particularly limited but preferably up to 4.0. Preferably, the material has a refractive index ranging from 1.9 to 4.0.

Particularly suitable materials are metal compounds and preferred are compounds where the metal is selected from zinc (Zn), titanium (Ti), zirconium (Zr) or a combination thereof. Preferably, the metal compound is (or at least comprises) a metal oxide such as titanium dioxide (TiO₂), zinc oxide (ZnO), zirconium dioxide (ZrO₂) or a combination thereof. In addition, the particulate whitening agent can also comprise non-metal oxides such as strontium titanate and zinc sulfide.

In a preferred embodiment, the particulate whitening agent comprises metal oxides, non-metal oxides or a combination thereof in an amount of at least 50% by weight of the whitening agent and more preferably at least 70%, more preferably still from 80 to 100% and most preferably from 85 to 95%. In an especially preferred embodiment, the particulate whitening agent is at least 50% by weight titanium dioxide, and most preferably, from 60 to 100% by weight titanium dioxide, based on total weight of the whitening agent and including all ranges subsumed therein. In another especially preferred embodiment, the particulate whitening agents are slightly soluble or insoluble in water, but most preferably, insoluble in water.

In a preferred embodiment, the particulate whitening agents are composite particles. The refractive index of a composite particle comprising more than one material can be calculated based on the refractive indices and volume fractions of the constituents using effective medium theory, as is described for example in WO 2009/023353.

The composite particle comprises a first component core and a second component coating. Typically, the core of the composite particle comprises a material suitable to physically and immediately improve characteristics of teeth and especially whiten teeth. In order to provide excellent whitening effect, the material is preferred to have a high refractive index of at least 1.9, more preferably at least 2.0, even more preferably at least 2.2, even more preferably still at least 2.4 and most preferably at least 2.5. The maximum refractive index of the material is not particularly limited but preferably up to 4.0. Preferably, the material has a refractive index ranging from 1.9 to 4.0.

Particular suitable materials are metal compounds and preferred are compounds where the metal is selected from zinc (Zn), titanium (Ti), zirconium (Zr) or a combination thereof. Preferably, the metal compound is (or at least comprises) a metal oxide such as titanium dioxide (TiO₂), zinc oxide (ZnO), zirconium dioxide (ZrO₂) or a combination thereof. In addition, the core of the composite particle can also comprise non-metal oxides such as strontium titanate and zinc sulfide.

The core of the composite particle typically makes up from 3 to 98%, and preferably from 6 to 65%, and most preferably from 10 to 55% by weight of the composite particle, based on total weight of the composite particle and including all ranges subsumed therein. In a preferred embodiment, the core comprises metal oxides, non-metal oxides or a combination thereof in an amount of at least 50% by weight of the core and more preferably at least 70%, more preferably still from 80 to 100% and most preferably from 85 to 95%. In an especially preferred embodiment, the core is at least 50% by weight titanium dioxide, and most preferably, from 60 to 100% by weight titanium dioxide, based on total weight of the first component core.

The second component coating comprises material suitable to adhere to tooth enamel, dentin or both. Typically the coating material comprises the element calcium, and optionally, other metals like potassium, sodium, aluminium, magnesium as well as mixtures thereof whereby such optional metals are provided as, for example, sulphates, lactates, oxides, carbonates or silicates. Optionally, the coating material may be aluminium oxide or silica. In a preferred embodiment, the coating material is suitable to provide a biological or chemical improvement to teeth which is long term (e.g., results in hydroxyapatite formation). Preferably, the coating employed comprises at least 50% by weight elemental calcium, and most preferably, at least 65% by weight elemental calcium based on total weight of metal in the coating. In an especially preferred embodiment, the metal in the coating is from 80 to 100% by weight of elemental calcium, based on total weight of metal in the second component coating and including all ranges subsumed therein. In another especially preferred embodiment, the core and the coating are slightly soluble or insoluble in water, but most preferably, insoluble in water.

In an especially desired embodiment, the second component coating can comprise, for example, calcium phosphate, calcium oxide, calcium carbonate, calcium hydroxide, calcium sulphate, calcium carboxymethyl cellulose, calcium alginate, calcium salts of citric acid, calcium silicate, mixture thereof or the like. In another desired embodiment, the calcium source in the coating comprises calcium silicate.

In yet another preferred embodiment, the coating can comprise the element calcium which originates from insoluble calcium silicate, present as the composite material calcium oxidesilica (CaO-SiO₂) as described in intemational patent applications published as WO 2008/015117 and WO 2008/068248.

When a calcium silicate composite material is employed as coating, the ratio of calcium to silicon (Ca:Si) may be from 1:10 to 3:1. The Ca:Si ratio is preferably from 1:5 to 3:1, and more preferably, from 1:3 to 3:1, and most preferably, from about 1:2 to 3:1. The calcium silicate may comprise mono-calcium silicate, bi-calcium silicate, or tri-calcium silicate whereby ratios of calcium to silicon (Ca:Si) should be understood to be atom ratios.

Usually, at least 30% of the outer surface area of the first component core is coated with the second component coating, preferably at least 50% of the core is coated with the coating, most preferably, from 70 to 100% of the outer surface area of the first component core is coated with the second component coating.

In an especially preferred embodiment, the particulate whitening agent is titanium dioxide coated with calcium silicate.

The particulate whitening agent according to the present invention can be of different sizes and shapes. The particles may be of spherical, platelet or irregular shape form. The diameter of the particulate whitening agent is often from 10 nm to less than 50 microns, and preferably, from 75 nm to less than 10 microns. In an especially preferred embodiment, the diameter of particles is from 100 nm to 5 microns, including all ranges subsumed therein. Particle size can be measured, for example, by dynamic light scattering (DLS). For composite particles, in a preferred embodiment, at least 40%, and preferably, at least 60%, and most preferably, from 75 to 99.5% of the diameter of the composite particle is the core, including all ranges subsumed therein.

The oral care composition of the present invention may comprise a single benefit agent or a mixture of two or more benefit agents. Typically, the benefit agent is present in an amount from 0.25 to 60%, and more preferably, from 0.5 to 40%, and most preferably, from 1 to 30% by total weight of the oral care composition and including all ranges subsumed therein.

When the benefit agent is incorporated into the oral care composition, the relative weight ratio of the water insoluble and/or slightly soluble calcium source to benefit agent may range from 1:10 to 30:1, preferably from 1:5 to 10:1, most preferably from 1:3 to 5:1.

The oral care composition of the present invention is found to be effective in blocking dentinal tubules to reduce tooth sensitivity. Without wishing to be bound by theory the present inventors believe that this may be because the water insoluble and/or slightly soluble calcium source reacts with the phosphate source to form calcium phosphate that may have an affinity for dentine and/or enamel of teeth. The tubule blockage enhancer of the present invention may further aid the deposition of the calcium source onto dentine and/or into dentinal tubules to induce *in situ* generation of calcium phosphate that occludes the dentinal tubules and/or their exposed open ends, which enhances the tubule blockage efficacy. When benefit agents are present in the oral care composition, the remineralization of the calcium source around the benefit agents further helps the retention of those benefit agents on tooth surfaces by enhancing their resistance to shear force.

The oral care composition of the present invention may contain a variety of other ingredients which are common in the art to enhance physical properties and performance. These ingredients include preservatives, pH-adjusting agents, sweetening agents, particulate abrasive materials, polymeric compounds, buffers and salts to buffer the pH and ionic strength of the compositions, and mixtures thereof. Such ingredients typically and collectively make up less than 20% by weight of the composition, and preferably, from 0.0 to 15% by weight, and most preferably, from 0.01 to 12% by weight of the composition, including all ranges subsumed therein.

The oral care composition of this invention is for use in benefiting teeth of an individual comprising applying in the composition to at least one surface of the teeth of an individual, said benefits includes reduced sensitivity, remineralization and combinations thereof. The oral care composition of this invention may additionally or alternatively be for use as a medicament and/or used in the manufacture of a medicament for providing an oral care benefit as described herein, such as for reducing sensitivity of the teeth of an individual. Alternatively and preferably, the use is non-therapeutic.

In a preferred embodiment, the oral care composition is a monophase anhydrous composition. The composition is substantially free of water to prevent the premature reaction between the water insoluble and/or slightly soluble calcium source and the phosphate source.

In another preferred embodiment, the oral care composition is a dual-phase composition comprising a calcium phase and a phosphate phase, wherein the water insoluble and/or slightly soluble calcium source and the tubule blockage enhancer are present in the calcium phase and the phosphate source is present in the phosphate phase. The two phases are physically separate from one another by being in independent phases. The delivery of the two independent phases to the teeth may be simultaneous or sequential. In a preferred embodiment, the phases are delivered simultaneously. When a dual-phase oral care composition is desired, water may act as a carrier (along with thickeners and/or additional carriers herein described) and make up the balance of each phase in the dual-phase composition.

When a dual-phase composition is used, the calcium phase and the phosphate phase should not come into contact with each other until dispensed for use. In use, it is preferably to combine the two phases to form a mix prior to their application to the teeth. Typically, the calcium phase and the phosphate phase are used at weight ratio from 20:80 to 80:20, more preferably from 35:65 to 65:35, and most preferably at a 50:50 weight ratio.

Typically, the dual-phase composition is delivered by a dual-tube having a first compartment for calcium phase and a second compartment for phosphate phase, which allows for coextrusion of the two phases.

In a preferred embodiment, such a dual-tube has one of the compartments surrounding the other. In such embodiments, one phase is present as a sheath, surrounding the other phase in the core. In an especially preferred embodiment, the core is the calcium phase and the sheath is the phosphate phase.

In another preferred embodiment, such a dual-tube has the two compartments side by side within the same tube. In such embodiments, the two phases are extruded from the tube as one, such extrusion being termed "contact extrusion". A pump head may be used in such a dual-tube for squeezing the two phases from the tube as one.

The dual-phase oral care composition may be a gel composition that comprises two independent gel phases, the first is the calcium phase and the second is the phosphate phase. The means of delivery may involve a cotton rod, or a tray, onto which the calcium phase and the phosphate phase are applied, prior to the tray being placed in contact with the teeth.

The oral care composition of the present invention is prepared by conventional methods of making oral care compositions. Such methods include mixing the ingredients under moderate shear and atmospheric pressure.

Typically the composition will be packaged. In tooth paste or gel form, the composition may be packaged in a conventional plastic laminate, metal tube or a single compartment dispenser. The same may be applied to dental surfaces by any physical means, such as a toothbrush, fingertip or by an applicator directly to the sensitive area. In liquid mouthwash form the composition may be packaged in a bottle, sachet or other convenient container.

The composition can be effective even when used in an individual's daily oral hygiene routine. For example, the composition may be brushed onto the teeth. The composition may, for example, be contacted with the teeth for a time period of one second to 20 hours. More preferably from 1 s to 10 hours, more preferably still from 10 s to 1 hour and most preferably from 30 s to 5 minutes. The composition may be used daily, for example for use by an individual once, twice or three times per day. When the oral care composition is a dual-phase composition, the two phases of the composition are mixed during application. The mixed phases are typically left on the teeth for from 3 minutes to 10 hours, more preferably from 3 minutes to 8 hours. The application may be carried out one to five times monthly.

The following examples are provided to facilitate an understanding of the present invention. The examples are not provided to limit the scope of the claims.

### Examples

### Example 1

This example demonstrates the improved blockage of dentinal tubules by using water insoluble and/or slightly soluble calcium source in combination with a tubule blockage enhancer. All ingredients are expressed by amount of the total formulation, and as level of active ingredient.

**TABLE 1**

| Ingredient (Amount/g) | **Samples** | |
|---|---|---|
| | **1** | **2** |
| Calcium carbonate^{a} | 0.5 | 0.5 |
| Calcium dihydrogen phosphate | -- | 0.1 |
| Sodium dihydrogen phosphate | 0.11 | 0.11 |
| Trisodium phosphate | 0.13 | 0.13 |

| | | |
|---|---|---|
| a) Commercially available microchalk (CaCO₃) with a particle size between 0.6 to 1.2 microns from Omya (Omyacare HP 900 OG) | | |

### Methods

To evaluate the blockage efficacy of dentinal tubules, the test sample was mixed with 10 mL water to make a slurry and used immediately

Human dentine disks were eroded by 37% phosphoric acid for 1 min, then they were treated with different slurries via brushing following the same protocol. The dentine disks were brushed with the slurry under a tooth brushing machine equipped with toothbrushes. The load of the tooth brushing was 170 g +/-5 g and the automatic brushing operated at a speed of 150 rpm. After brushing for 1 min, the dentine disks were soaked in toothpaste slurry for 1 min. Then the dentine disks were placed in de-ionized (DI) water and agitated on a flatbed shaker at 150 rpm for 10 strokes. After that, the dentine disks were soaked in simulated oral fluid (SOF) under the condition of a shaking water bath at 37°C and 60.0 rpm. After soaking for more than 3 hours, the dentine disks were brushed with the slurry by machine using the same procedure as in the first step. The brushing was repeated two to three times for one day, then the dentine disks were kept in SOF overnight(>12 hours) in a shaking water bath at 37°C to mimic oral environment. The dentine samples were brushed for 3 times.

Simulated oral fluid was made by combining the ingredients in Table 2:

**TABLE 2**

| Ingredient | Amount/g |
|---|---|
| NaCl | 16.07 |
| NaHCO₃ | 0.7 |
| KCI | 0.448 |
| K₂HPO₄*3H₂O | 3.27 |
| MgCl₂*6H₂O | 0.0622 |
| 1M HCI | 40 mL |
| CaCl₂ | 0.1998 |
| Na₂SO₄ | 0.1434 |
| Buffer | Adjust pH to 7.0 |
| Water | Balance to 2 L |

### Scoring Standard for Tubules Blockage

Regardless of the original shape of the dentine disks, a square (with a size of 4mm x 4mm) is selected and one image is captured under 50x magnification. Within this square, five spots (each with a size of 150 µm × 150 µm, one in the middle, and one in every corner) are selected and observed under 1000x magnification. The blockage of tubules are accessed following the standards in Table 3. The measurement is repeated twice for each sample.

**TABLE 3**

| **Score** | **Tubules Blockage** |
|---|---|
| 0 | All dentinal tubules are open. |
| 1 | <20% of dentinal tubules are fully blocked. |
| 2 | 20 to 50% of dentinal tubules are fully blocked. |
| 3 | 50 to 80% of dentinal tubules are fully blocked. |
| 4 | 80-100% of dentinal tubules are fully blocked. |
| 5 | All dentinal tubules are fully blocked. |

### Results

After 3 brushings, SEM (Scanning Electron Microscopy, Hitachi S-4800) images of the dentine disks were taken. The images were analyzed and scored. The results are summarized in Table 4 (error represents standard error for duplicate measurements).

**TABLE 4**

| | | |
|---|---|---|
| Tubules Blockage Score | **Samples** | |
| | **1** | **2** |
| | 2.6 ± 0.17 | 4.2 ± 0.35 |

It can be seen that the dentinal tubules of dentine disks treated with Sample 2 were filled significantly better (p<0.05) compared to Sample 1 comprising no tubule blockage enhancer. For Sample 1, some of the dentinal tubules were blocked by mineral deposition, but still lots of dentinal tubules were open. While for Sample 2, most of the tubules were extensively blocked and sealed.

## Claims

1. An oral care composition comprising:
a) a water insoluble and/or slightly soluble calcium source;
b) a tubule blockage enhancer selected from calcium dihydrogen phosphate, calcium sulfate hemihydrates or mixtures thereof;
c) a phosphate source; and
d) a physiologically acceptable carrier;
wherein the calcium source is selected from calcium carbonate, calcium aluminate, calcium oxalate, aluminium calcium silicate, calcium hydroxide, calcium glycerophosphate, calcium oxide, calcium sulfate, calcium carboxymethyl cellulose, calcium alginate, calcium salts of citric acid or mixtures thereof;
wherein the phosphate source is trisodium phosphate, monosodium dihydrogen phosphate, disodium hydrogen phosphate, ammonium phosphate, diammonium hydrogen phosphate, ammonium dihydrogen phosphate, tripotassium phosphate, monopotassium dihydrogen phosphate, dipotassium hydrogen phosphate or a mixture thereof; and
wherein the calcium source and the tubule blockage enhancer are present in a weight ratio from 20:1 to 1:5.

2. The oral care composition according to claim 1, wherein the calcium source comprises at least 80% by weight of calcium carbonate based on the total weight of the calcium source.

3. The oral care composition according to claim 1 or claim 2, wherein the calcium source is calcium carbonate.

4. The oral care composition according to any of the preceding claims, wherein the calcium source has a particle size of 5 micron or less, preferably from 0.01 to 3 microns, more preferably from 0.01 to 1.5 microns.

5. The oral care composition according to any of the preceding claims, wherein the tubule blockage enhancer is calcium dihydrogen phosphate.

6. The oral care composition according to any of the preceding claims, wherein the calcium source is present in an amount from 0.1 to 80%, preferably 0.2 to 50% by weight of the composition.

7. The oral care composition according to any of the preceding claims, wherein the calcium source and the tubule blockage enhancer is present in a weight ratio from 15:1 to 1:3, preferably from 10:1 to 1:1.

8. The oral care composition according to any of the preceding claims, wherein the phosphate source is present in an amount from 0.1 to 40%, preferably from 0.5 to 30% by weight of the composition.

9. The oral care composition according to any of the preceding claims, wherein the calcium source and the phosphate source are present in a weight ratio from 10:1 to 1:5, preferably from 5:1 to 1:3.

10. The oral care composition according to any of the preceding claims, wherein the composition further comprises a benefit agent, preferably a particulate whitening agent.

11. The oral care composition according to claim 10, wherein the particulate whitening agent is a composite particle, preferably titanium dioxide coated with calcium silicate.

12. The oral care composition according to any of the preceding claims, wherein the oral care composition is a monophase anhydrous composition.

13. The oral care composition according to any one of claims 1 to 11, wherein the composition is a dual-phase composition comprising a calcium phase and a phosphate phase, wherein the water insoluble and/or slightly soluble calcium source and the tubule blockage enhancer are present in the calcium phase, and the phosphate source is present in the phosphate phase.

14. An oral care composition according to any one of claims 1 to 13 for use in reducing sensitivity and/or remineralizing of teeth.

## Patentansprüche

1. Mundpflegezusammensetzung, umfassend:
a) eine wasserunlösliche und/oder schwach lösliche Calciumquelle;
b) einen Tubulus-Blockade-Verstärker, ausgewählt aus Calciumdihydrogenphosphat, Calciumsulfat-Hemihydraten oder Mischungen davon;
c) eine Phosphatquelle; und
d) einen physiologisch verträglichen Träger;
wobei die Calciumquelle aus Calciumcarbonat, Calciumaluminat, Calciumoxalat, Aluminiumcalciumsilikat, Calciumhydroxid, Calciumglycerophosphat, Calciumoxid, Calciumsulfat, Calciumcarboxymethylcellulose, Calciumalginat, Calciumsalzen von Citronensäure oder Mischungen davon ausgewählt ist;
wobei die Phosphatquelle Trinatriumphosphat, Mononatriumdihydrogenphosphat, Dinatriumhydrogenphosphat, Ammoniumphosphat, Diammoniumhydrogenphosphat, Ammoniumdihydrogenphosphat, Trikaliumphosphat, Monokaliumdihydrogenphosphat, Dikaliumhydrogenphosphat oder eine Mischung davon ist und wobei die Calciumquelle und der Tubulus-Blockade-Verstärker in einem Gewichtsverhältnis von 20:1 bis 1:5 vorliegen.

2. Mundpflegezusammensetzung nach Anspruch 1, wobei die Calciumquelle mindestens 80 Gewichts-% Calciumcarbonat, bezogen auf das Gesamtgewicht der Calciumquelle, umfasst.

3. Mundpflegezusammensetzung nach Anspruch 1 oder Anspruch 2, wobei die Calciumquelle Calciumcarbonat ist.

4. Mundpflegezusammensetzung nach irgendeinem der vorhergehenden Ansprüche, wobei die Calciumquelle eine Partikelgröße von 5 µm oder weniger, vorzugsweise von 0,01 bis 3 µm, bevorzugter von 0,01 bis 1,5 µm, aufweist.

5. Mundpflegezusammensetzung nach irgendeinem der vorhergehenden Ansprüche, wobei der Tubulus-Blockade-Verstärker Calciumdihydrogenphosphat ist.

6. Mundpflegezusammensetzung nach irgendeinem der vorhergehenden Ansprüche, wobei die Calciumquelle in einer Menge von 0,1 bis 80 Gewichts-%, vorzugsweise von 0,2 bis 50 Gewichts-% der Zusammensetzung vorliegt.

7. Mundpflegezusammensetzung nach irgendeinem der vorhergehenden Ansprüche, wobei die Calciumquelle und der Tubulus-Blockade-Verstärker in einem Gewichtsverhältnis von 15:1 bis 1:3, vorzugsweise von 10:1 bis 1:1, vorliegen.

8. Mundpflegezusammensetzung nach irgendeinem der vorhergehenden Ansprüche, wobei die Phosphatquelle in einer Menge von 0,1 bis 40 Gewichts-%, vorzugsweise von 0,5 bis 30 Gewichts-% der Zusammensetzung vorliegt.

9. Mundpflegezusammensetzung nach irgendeinem der vorhergehenden Ansprüche, wobei die Calciumquelle und die Phosphatquelle in einem Gewichtsverhältnis von 10:1 bis 1:5, vorzugsweise von 5:1 bis 1:3, vorliegen.

10. Mundpflegezusammensetzung nach irgendeinem der vorhergehenden Ansprüche, wobei die Zusammensetzung ferner ein Pflegemittel, vorzugsweise einen teilchenförmigen Weißmacher, umfasst.

11. Mundpflegezusammensetzung nach Anspruch 10, wobei der teilchenförmige Weißmacher ein Verbundpartikel, vorzugsweise ein mit Calciumsilikat beschichtetes Titandioxid, ist.

12. Mundpflegezusammensetzung nach irgendeinem der vorhergehenden Ansprüche, wobei die Mundpflegezusammensetzung eine einphasige wasserfreie Zusammensetzung ist.

13. Mundpflegezusammensetzung nach irgendeinem der Ansprüche 1 bis 11, wobei die Zusammensetzung eine zweiphasige Zusammensetzung ist, umfassend eine Calciumphase und eine Phosphatphase, wobei die wasserunlösliche und/oder schwach lösliche Calciumquelle und der Tubulus-Blockade-Verstärker in der Calciumphase und die Phosphatquelle in der Phosphatphase vorliegen.

14. Mundpflegezusammensetzung nach irgendeinem der Ansprüche 1 bis 13 zur Verwendung bei der Verringerung der Empfindlichkeit und/oder der Remineralisierung der Zähne.

## Revendications

1. Composition pour le soin oral comprenant :
a) une source de calcium insoluble et/ou légèrement soluble dans l'eau ;
b) un promoteur de blocage de tubule choisi parmi du dihydrogénophosphate de calcium, des hémihydrates de sulfate de calcium ou mélanges de ceux-ci ;
c) une source de phosphate ; et
d) un support physiologiquement acceptable ;
dans laquelle la source de calcium est choisie parmi le carbonate de calcium, aluminate de calcium, oxalate de calcium, silicate d'aluminium calcium, hydroxyde de calcium, glycérophosphate de calcium, oxyde de calcium, sulfate de calcium, carboxyméthylcellulose de calcium, alginate de calcium, sels de calcium d'acide citrique ou mélanges de ceux-ci ;
dans laquelle la source de phosphate est le phosphate de trisodium, dihydrogénophosphate de monosodium, hydrogénophosphate de disodium, phosphate d'ammonium, hydrogénophosphate de diammonium, dihydrogénophosphate d'ammonium, phosphate de tripotassium, dihydrogénophosphate de monopotassium, hydrogénophosphate de dipotassium ou un mélange de ceux-ci ; et
dans laquelle la source de calcium et le promoteur de blocage de tubule sont présents dans un rapport de masse de 20:1 à 1:5.

2. Composition pour le soin oral selon la revendication 1, dans laquelle la source de calcium comprend au moins 80 % en masse de carbonate de calcium sur la base de la masse totale de la source de calcium.

3. Composition pour le soin oral selon la revendication 1 ou revendication 2, dans laquelle la source de calcium est le carbonate de calcium.

4. Composition pour le soin oral selon l'une quelconque des revendications précédentes, dans laquelle la source de calcium présente une taille de particule de 5 microns ou inférieure, de préférence de 0,01 à 3 microns, encore mieux de 0,01 à 1,5 microns.

5. Composition pour le soin oral selon l'une quelconque des revendications précédentes, dans laquelle le promoteur de blocage de tubule est le dihydrogénophosphate de calcium.

6. Composition pour le soin oral selon l'une quelconque des revendications précédentes, dans laquelle la source de calcium est présente dans une quantité de 0,1 à 80 %, de préférence de 0,2 à 50 % en masse de la composition.

7. Composition pour le soin oral selon l'une quelconque des revendications précédentes, dans laquelle la source de calcium et le promoteur de blocage de tubule sont présents dans un rapport de masse de 15:1 à 1:3, de préférence de 10:1 à 1:1.

8. Composition pour le soin oral selon l'une quelconque des revendications précédentes, dans laquelle la source de phosphate est présente dans une quantité de 0,1 à 40 %, de préférence de 0,5 à 30 % en masse de la composition.

9. Composition pour le soin oral selon l'une quelconque des revendications précédentes, dans laquelle la source de calcium et la source de phosphate sont présentes dans un rapport de masse de 10:1 à 1:5, de préférence de 5:1 à 1:3.

10. Composition pour le soin oral selon l'une quelconque des revendications précédentes, dans laquelle la composition comprend de plus un agent bénéfique, de préférence un agent de blanchiment particulaire.

11. Composition pour le soin oral selon la revendication 10, dans laquelle l'agent de blanchiment particulaire est une particule composite, de préférence du dioxyde de titane revêtu de silicate de calcium.

12. Composition pour le soin oral selon l'une quelconque des revendications précédentes, dans laquelle la composition pour le soin oral est une composition anhydre de monophase.

13. Composition pour le soin oral selon l'une quelconque des revendications 1 à 11, dans laquelle la composition est une composition à phase double comprenant une phase de calcium et une phase de phosphate, dans laquelle la source de calcium insoluble et/ou légèrement soluble dans l'eau et le promoteur de blocage de tubule sont présents dans la phase de calcium, et la source de phosphate est présente dans la phase de phosphate.

14. Composition pour le soin oral selon l'une quelconque des revendications 1 à 13 pour une utilisation dans la réduction de la sensibilité et/ou la reminéralisation des dents.
